# EUROPEAN PATENT SPECIFICATION

(11) **EP 1 835 053 B1**
(45) Date of publication and mention of the grant of the patent: **16.03.2011**
(21) Application number: 06005523.3
(22) Date of filing: 17.03.2006
(51) Int. Cl.: C30B 29/10, C30B 29/58, A61K 35/56, C30B 7/00

(54) **Synthetic nacre, method of manufacture and apparatus therefor**
Synthetische Perlmutter, Verfahren und Vorrichtung zu ihrer Herstellung
Nacre synthétique, méthode et appareil pour sa fabrication

(43) Date of publication of application: 19.09.2007
(73) Proprietor: Universität Bremen, 28359 Bremen (DE)
(72) Inventor: Fritz, Monika, 28870 Ottersberg (DE); Treccani, Laura, 28205 Bremen (DE); Heinemann, Fabian, 28201 Bremen (DE); Grathwohl, Georg, 28207 Bremen (DE)
(74) Representative: Steinecke, Peter

(56) References cited:
- US-A- 5 755 787
- BECKER ET AL.: "A high-throughput crystallization device to study biomineralization in vitro" MATER.RES.SOC.SYMP.PROC, vol. 873E, 2005, pages K12.1.1-K12.1.10, XP008067027
- MANJUBALA ET AL: "Mineralisation of chitosan scaffolds with nano-apatite formation by double diffusion technique" ACTA BIOMATERIALIA, ELSEVIER, AMSTERDAM, NL, vol. 2, no. 1, January 2006 (2006-01), pages 75-84, XP005221579 ISSN: 1742-7061
- TANG ET AL: "Nanostructured artificial nacre" NATURE MATERIALS, vol. 2, June 2003 (2003-06), pages 413-418, XP002391848 London GB
- MANOLI ET AL: "Crystallization of calcite on chitin" J.CRYSTAL GROWTH, vol. 182, 1997, pages 116-124, XP002391849 ELSEVIER AMSTERDAM NL
- GRASSMANN O ET AL: "Biomimetic nucleation and growth of CaCO3 in hydrogels incorporating carboxylate groups" BIOMATERIALS, ELSEVIER SCIENCE PUBLISHERS BV., BARKING, GB, vol. 25, no. 2, January 2004 (2004-01), pages 277-282, XP004469618 ISSN: 0142-9612
- MUKKAMALA ET AL: "Modelling calcium carbonate biomineralisation processes" JOURNAL OF INORGANIC BIOCHEMISTRY, ELSEVIER, vol. 100, no. 5-6, May 2006 (2006-05), pages 1128-1138, XP005419655 ISSN: 0162-0134
- MUZZARELLI C ET AL: "Natural and artificial chitosan-inorganic composites" JOURNAL OF INORGANIC BIOCHEMISTRY, ELSEVIER, vol. 92, no. 2, 11 November 2002 (2002-11-11), pages 89-94, XP004702939 ISSN: 0162-0134

## Description

### Field of the invention

The present invention provides self-organizing synthetic organic composits of high organized structure which is particular useful as sustainable bioceramic. More specifically, the present invention pertains to biogenic polymer/mineral compositions obtained by specific crystallization in vitro. In particular, synthetic nacre is provided. Furthermore, the present invention generally relates to methods of manufacturing crystals naturally found in nature. In particular, it relates to a method of manufacturing nacre and derivatives thereof. The present invention further concerns a device, appropriate for crystallization of aragonite to occur inside.

### Background of the invention

Biogenic calcium carbonate is besides its universal presence in nature an interesting material to be used for sustainable modem human purposes and it is in the form of nacre suited to serve as a pattern for advanced recyclable bioceramics. Biominerals, biogenic polymer/mineral composites, produced by many organisms are characterized by high functionality and superior properties compared to their biogenic counterparts. Another essential feature of materials produced by biomineralization is their highly controlled hierarchical organization down to the nano- and molecular scale. Molecular structuring is achieved by a high level of physico-chemical and spatial control over the precipitation process caused by organic molecules interacting with the mineral ions.

The amazing properties of the biomineral nacre are due to the highly organized structure guided by the organic material, which is incorporated in the mineral phase aragonite, a calcium carbonate polymorph; see for example Nakahara, Venus 38 (1979), 205-211; Fritz and Morse, Curr. Opin. Cell Biol. 3 (1998), 55-62. The biogenic polymer/mineral composite nacre is grown by a self-organization process, where a few weight percent of organic material governs the specific crystallization of the calcium carbonate polymorph aragonite. The thus developed material shows a dense packing of thin layers (500nm) of mineral interdispersed by a few nanometer of organics, acting like a glue to improve the mechanical properties of this biogenic ceramic by making it non-brittle. It was shown that several proteins influence the crystallization of calcium carbonate, some of them are attached to chitin, while others are water-soluble.

The organic material (see for example Hare and Abelson, Carnegie Inst. Wash. Yearbook 64 (1965), 223-234) is involved in nucleation, polymorph selection and morphology - from nanoscale to macroscale - at a degree, which is far beyond today's technological capabilities. This high degree of control leads to a final product, which is a highly ordered organic - inorganic composite of extraordinary toughness; see for example Jackson et al., J. Mat. Sci. 25 (1990), 3173-3178.

In nature, the growth of nacre takes place in a preformed three dimensional extracellular matrix, soaked with the extrapallial fluid consisting of defined ionic concentrations (see for example Crenshaw, Bio. Bull. 143 (1972), 506-512) and dissolved proteins, which are secreted by the mantle epithelium of the animal. For the researcher, this organic material is accessible after demineralization, leaving the water-soluble matrix fraction and the water-insoluble, interlamellar, organic matrix (short: insoluble matrix). The interaction of these two matrix fractions during the growth of the material is theoretically not fully understood. Many studies have been performed on water-soluble matrix proteins. It is known that many of them interact strongly with growing CaCO₃ crystals. The entity of the water-soluble matrix is able to suppress crystallization in solution, but also to promote the growth of (001) oriented aragonite on (1014) calcite surfaces; see for example Thompson et al., Biophysical J. 79 (2000), 3307-3312. The insoluble matrix is thought to act as a nucleation surface and predefined mold, determining size and orientation of the crystals. Individual sheets of the insoluble matrix have a thickness of approximately 40 nm (abalone nacre) with a core of highly ordered β-chitin (see for example Weiss et al., Chem. Mat. 14 (2002), 3252-3259; Weiner and Traub, FEBS Lett. 111 (1980), 311-316) and a periphery consisting of silk-fibroin-like protein; see for example Addadi and Weiner, Angew. Chem. Int. Ed. Engl. 31 (1992), 153-169. It has been shown, that the insoluble matrix is permeable for ions and macromolecules; see for example Schäffer et al., Chem. Mat. 9 (1997), 1731-1740.

Manjubala et al., Acta Biomaterialia 2 (2006), 75-84 describe the mineralization of chitosan scaffolds with nano-apatite formation by double diffusion technique in order to provide a suitable substrate for cell attachment and migration in bone tissue engineering.
Tang et al., Nature Materials 21 (2003), 423-418 describe the preparation of artificial nacre by sequential deposition of montmonillonite clay platelets and polyelectrolytes by sequential absorption of organic and inorganic dispersions.

With respect to methods of manufacturing of crystal hitherto available, either synthetic or not, there are several disadvantages. Concerning for example the widely used ammonium carbonate method (see for example Addadi and Weiner, Proc. Natl. Acad. Sci. USA, 82 (1985), 4110-4114), it exhibits one major drawback, namely the pH rising over 9.0, a value where CaCO₃ precipitation is quite rapid and surface charges of the insoluble matrix may differ from the native state (the pH-value of the EPF is about 7.5; see for example Crenshaw, Bio. Bull. 143 (1972), 506-512). In addition, common double diffusion methods with two vessels separated by the insoluble matrix seem also inappropriate, because the precipitation of CaCO₃ is accompanied with a decrease in pH, which changes surface properties of the matrix and leads in combination with the non-constant concentrations to a non-constant precipitation rate.

Thus, there is a need in the art for means and methods of manufacturing sustainable biominerals such as self-organizing nacre *in vitro.* These means and methods for manufacturing are provided by the present invention.

### Summary of the invention

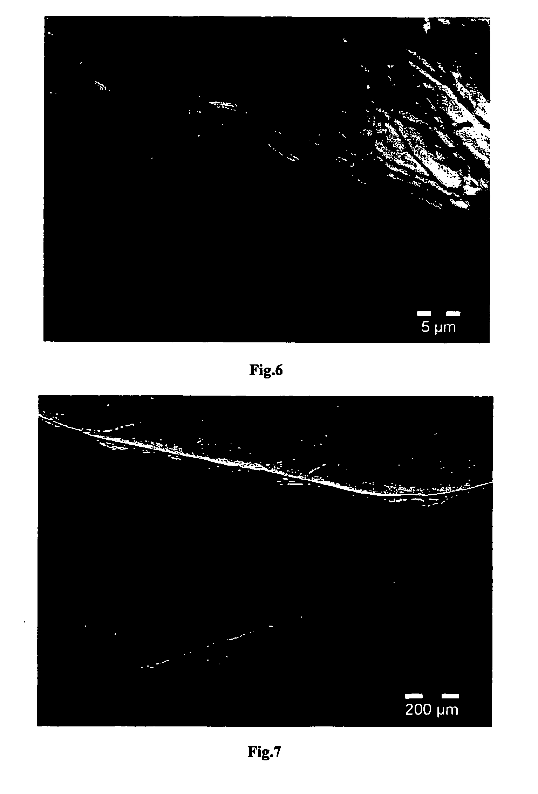

The present invention overcomes the drawbacks of the prior art. Experiments performed in accordance with the present invention revealed that by remineralizing the insoluble matrix of abalone nacre in a modified double diffusion device where diffusing calcium carbonate-salt solutions were constantly renewed by a pump thus maintaining a constant ionic level nacre like crystals can be obtained. In further experiments, crystallization was carried out on for example cellulose membranes and surprisingly it could be shown for the first time that the insoluble matrix induces the growth of flat and oriented aragonite crystals on the matrix surface. In some of the experiments with the insoluble matrix, growth of parallel sheets of aragonite platelets inside the membrane was observed, looking identical to natural nacre; see also Example 5, and Figures 6 and 7.

Thus, the present invention is directed to a method, as set forth in claim 1, of manufacturing crystals normally found in nature. More specifically, the present invention provides a method for growing sustainable polymer/mineral composits, in particular nacre or derivatives thereof comprising (a) providing a two- or three-dimensional template which is permeable for ions and macromolecules in double diffusion; (b) subjecting the template to a continuous flow of a calcium ion containing solution on one side and a carbonate ion containing solution on the other side of the template; under conditions allowing nucleating crystals.

Furthermore, the present invention relates to a device, as set forth in claim 10 in which crystallization is realized, comprising a double diffusion crystallization box. The device comprises a double diffusion crystallization box, a template by which the box when closed is separated into two separate halves, a first and a second half, a template-holder, and solution inlet(s) and outlet(s) in each of the two halves of the box, and said device optionally may further comprise means for continuously pumping solutions through the two separated parts of the box, and optionally solution reservoirs.

It is an object of the present invention to provide biominerals, in particular synthetic nacre.

It is another object of the present invention to provide a composition or device comprising nacre obtainable by the method of the present invention. In particular, the sustainable polymer/mineral composits of the present invention, especially nacre, find application in paint, coatings for surfaces or coatings for medical devices like needles, syringes or pumps, ceramics, buildings, transportation systems like aircraft, ships and automobiles, and/or bone or dental implant.

### Brief description of the drawings

- **Fig. 1:**: represents in its upper images scanning electron microscopy (SEM) images of shell (*Haliotis laevigata*). Upper left side illustrates the transition between calcite (upper left corner) and nacre's aragonite (lower right corner) in the natural shell. Upper right side shows aragonite platelets in nacre. Lower images represent a schematic illustration of the distribution of mineral and organic material.

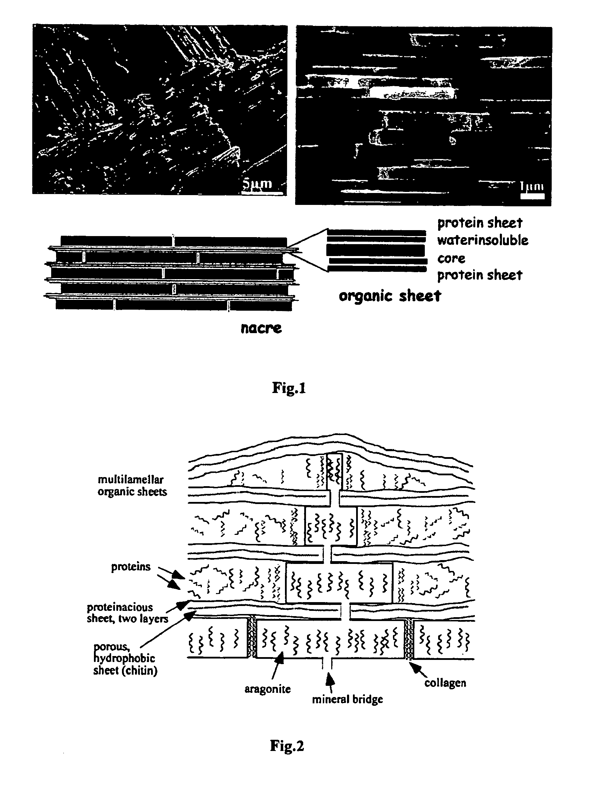

- **Fig. 2:**: illustrates schematics of the hypothesized nacre growth front. Multilamellar sheets containing water insoluble proteins and chitin are shown to be first deposited. Into this mold, aragonite plates are assumed to be crystallized. The involved water soluble proteins get incorporated into the aragonite plates as intracrystalline proteins or stay between the aragonite plates as intercrystalline proteins. As can be seen, in nacre, roughly hexagonal aragonite platelets (0.5 µm in height, 5-15 µm in diameter) are arranged in layers, separated by sheets of the so-called interlamellar organic matrix in vertical direction. The individual aragonite platelets are strongly oriented, such that the (001) plane of aragonite is parallel to the macroscopic laminar sheet of nacre; see also for example Fritz et al., Nature 371 (1994), 49-51.

- **Fig. 3:**: represents a Finite-Element-Method (FEM)-model and shows the overlapping mineral platelets in the layered nacre structure.
- **Fig. 4:**: shows the results of FEM calculation. Poisson's ratio of biopolymer is varied.
- **Fig. 5:**: illustrates schematically the double-diffusion crystallization box (opened). It shows an ion permeable membrane separating the constantly pumped carbonate and calcium ion containing solutions, which mix by diffusion through the membrane. The box comprises two flow channels per side, allowing for a better radial distribution of ions.
- **Fig. 6:**: illustrates a scanning electron microscopic image of a cross section of synthetic nacre.
- **Fig. 7:**: illustrates a scanning electron microscopic image of a crack in the synthetic nacre sample. The aragonite plates are visible within the three dimensional membrane.

### Detailed description of the invention

The present invention generally relates to a method of manufacturing biominerals in the form of self-organizing composites usually found in and unique for nature. In particular, it relates to a method of manufacturing nacre and derivatives thereof. In one aspect, the method of the present invention is based on providing a selectively permeable template (or matrix) being passed around by ionic solutions providing conditions allowing for self-organized crystal formation.

As discussed in the background section, biogenic calcium carbonate is one of the most interesting and industrial important polymer/mineral composite because of its for example corrosion and fracture resistance. For this reason, the method of the present invention employs ionic solutions which when coming in contact with organic material such as a water-insoluble organic matrix are capable of nucleating into a biogenic calcium carbonate crystal. Therefore, preferably calcium ion and carbonate ion containing solutions are employed in accordance with the present invention.

In order to provide the highly organized structure commonly found in nature for biominerals, a two- or three-dimensional template is used in the method of the present invention, which preferably reflects the three-dimensional extra-cellular matrix of for example nacre, and which is meant to act as nucleation surfaces.

Accordingly, in one embodiment the present invention relates to a method of manufacturing nacre or derivatives thereof comprising:
(a) providing a two- or three-dimensional template which is permeable for ions and macromolecules in double diffusion;
(b) subjecting the template to a continuous flow of a calcium ion containing solution on one side and a carbonate ion containing solution on the other side of the template; under conditions allowing nucleating crystals.

As demonstrated in the examples, biogenic polymer/mineral composites produced according to the method of the present invention possess a dense packing of the mineral plates and comprise the interaction of the organic polymer with the mineral plates, thus similar and even virtually identical to the most prominent biogenic polymer/mineral composite nacre; see Examples 3 and 4 as well as Figures 6 and 7. Accordingly, the present invention provides for the first time a method of manufacturing biogenic calcium carbonate crystals in vitro, which exhibit the useful properties of their natural counterparts. In particular, an easy to perform and reliable method for the manufacture of a biomineral is provided, which displays the unique properties of nacre. For this reason, the biogenic polymer/mineral composition or crystal produced in accordance with the method of the present invention is also referred to as "synthetic nacre".

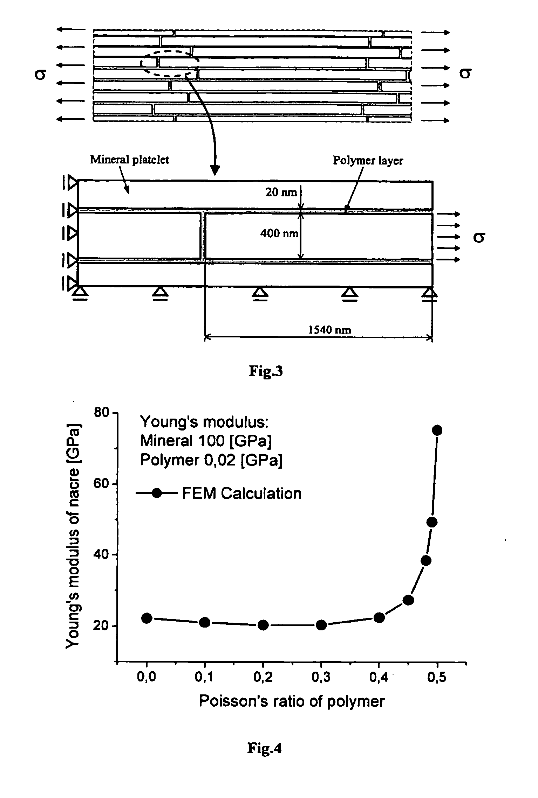

In context with the present invention, the term "nacre and/or derivative thereof" refers to biogenic polymer/mineral composites which preferably comprise a soft, corrosion-resistant water-insoluble organic matrix, a dense packing of mineral plates and interaction of the organic material with the mineral plates; see for example Fig. 3. Preferably, the term "nacre and/or derivative thereof" refers to the biogenic polymer/mineral composit nacre and to equivalent biominerals obtainable by the method of the present invention. The amazing properties of the biomineral nacre for example are due to the highly organized structure guided by the organic material, which is incorporated in the mineral phase aragonite, a calcium carbonate polymorph, and wherein the biogenic polymer/mineral composite nacre is grown by a self-organization process, where a few weight percent of organic material governs the specific crystallization of the calcium carbonate polymorph aragonite.

If not stated otherwise, the terms "template", "three dimensional matrix", "three dimensional template", or "membrane" are used interchangeably herein and refer to a kind of diaphragm separating two solutions of different ion compositions from each other, having a certain permeability to allow certain molecules to pass through, thereby allowing the mixture of the two solutions and the ions in the solution, respectively. For the purposes of the present invention said template is preferably comprised of an insoluble organic matrix similar as nacre. Without intending to be bound by theory, it is believed that said template acts as a nucleation surface and thus template for crystallization. For *in vitro* manufacturing of self-organized composites such as nacre organic matrix seems to be essential and it has been shown, that the insoluble matrix is permeable for ions and macromolecules; see for example Schäffer et al., Chem. Mat. 9 (1997), 1731-1740.

The terms "double diffusion device", "crystallization box" or "double diffusion crystallization box", refer to an apparatus for crystallization and may be used interchangeably herein.

Aragonite is a carbonate mineral, a polymorph of the mineral calcite, both having the chemical composition CaCO₃. Its structure differs from calcite and leads to a different crystal shape, an orthorhombic system, while calcite is trigonal oriented. Aragonite and calcite further differ in crystallography, and have widely different properties. Aragonite is harder and denser than calcite, having a higher specific gravity, since the atoms in aragonite are packed a little closer. They further differ in solubility, since aragonite is also more soluble in water than calcite. Aragonite occurs in several different geological and biological environments and is of biological interest, forming skeletal structures of many organisms. In contrast to the skeletons of humans and other vertebrates which are composed of phosphates, many shells and other exoskeletons of invertebrates are composed of aragonite, as well as most mollusc shells and modem corals. Nacre, also termed "mother of pearl", is one of the more spectacular forms of biologically deposited aragonite.

Accordingly, in a particularly preferred embodiment, the crystals manufactured by the method of the present invention are aragonite crystals.

As described in the appended examples, the method of the present invention employs a double diffusion device, where under constant composition (see also Becker and Epple, Mater. Res. Soc. Symp. Proc. 2005. 873E:K12.1.1-K12.1.10) of the crystallization components and by using a three dimensional template synthetic nacre is produced under atmospheric pressure and room temperature (ambient conditions). In the interior of the double diffusion device, from both sides a calcium ion containing solution such as CaCl₂ and a carbonate ion containing solution such as NaHCO₃, respectively, with constant mass flow rates are flown through which mix by diffusion through the membrane, i.e. form a CaCO₃-salt which precipitates. Macroscopic mineralization inside the three-dimensional template leading to a layered composite with the characteristic iridescent appearance (Fig. 6) is received.

Accordingly, in one embodiment of the method of the present invention, said calcium ion containing solution comprises a dissolved calcium salt, with a solubility product higher than calcium carbonate, preferably wherein said calcium ion containing solution comprises CaCl₂, and/or CaNO₃. Likewise preferred, the carbonate ion containing solution comprises a dissolved carbonate salt, with a solubility product higher than calcium carbonate, preferably wherein said carbonate ion containing solution comprises NaHCO₃ and/or Na₂CO₃. Most preferably, said calcium ion containing solution comprises CaCl₂ and/or CaNO₃, and/or said carbonate ion containing solution comprises NaHCO₃.

In another embodiment a calciumphosphate ion containing solution can be used in the method of the present invention, thereby allowing for the growth of crystals, applicable as for example bone substitution material.

Preferably, one or both of said calcium and carbonate ion containing solutions are either saturated or supersaturated. Most preferably, said calcium and carbonate ion containing solutions are solutions of CaCl₂ and NaHCO₃ supersaturated with respect to calciumcarbonate when mixed, which are to be used on the respective side of the double diffusion device to allow for crystallization.

When carrying out the method of the present invention the ionic strength, i.e. the composition of the participating ion solution is preferably intended to be kept constant for CaCl₂ and NaHCO₃. Thus, in a further preferred embodiment the calcium ion containing solution has a pH of about 7, and/or said carbonate ion containing solution has pH of about 8.

As discussed before, the growth of the biogenic polymer/mineral composite is governed by a two- or preferably three-dimensional organic matrix. For native nacre this matrix has been identified to comprise a soft, corrosion-resistant water-insoluble organic matrix with a core of chitin. For this reason, the method of the present invention preferably employs a template comprising chitin or an equivalent polymer. For example, template can consist of or comprise functionalized chitin or cellulose or any water insoluble substrate, which forms a three dimensional layered mold (matrix) providing the surface functionality for heterogeneous nucleation of aragonite. Crystal deposition on surfaces (heterogeneous crystallization) is used in comparison to precipitation from solution, because the energy barrier for the development of stable nuclei is lowered. A method is implemented to favour crystallization nucleated by a functionalized three-dimensional matrix.

In natural nacre the organic matrix (reviewed by Treccani et al., Biopolymers. 2003. Weinheim: Wiley-VCH Verlag GmbH, 289-321) acts as a glue between the mineral plates; see also Fig. 1. When the "glue" polymers are deformed at an external load, it is thought that the energy is dissipated; see for example Evans et al., J. Mater. Res. 16 (2001), 2475. Up to now, there has no single, purified protein been identified, which is responsible for the growth of aragonite instead of calcite. Just in the presence of mixtures of water-soluble proteins there was the observation for aragonite growth on calcite crystals; see for example Belcher et al., Nature 381 (1996), 56-58.

As mentioned, one important organic species in naturally occurring nacre is chitin; see for example Weiss et al., Chem. Mat. 14 (2002), 3252-3259. It is a hydrophobic polymer, which forms the core of the multilamellar organic sheet, which is the soft organic material preventing the mineral in the polymer/mineral composite from cracking (Fig. 2). It has been shown that chitin is very resistant against chemical degradation and it might protect nacre against corrosion in seawater and might even be the substance, which prevents nacre from being attacked by bacteria, algea and other "fouling organisms" in sea water. The chitin core itself is hydrophobic, but it binds acidic proteins such that the demineralised organic material, when all the water-soluble proteins are removed, is negatively charged. The negative charges are thought to be responsible for the interaction with the mineral. Thus, chitin and derivatives of chitin are the preferred components for the template used in the method of the present invention.

A further characteristic of the method of the present invention is a two- or three-dimensional template, which nucleates aragonite crystals from infiltrating the ions. The template should preferably be either the water-insoluble matrix of mussel, snail or any other chitin containing protecting armament of a sea water animal, or functionalized chitin from the same source, or any other natural, functionalized polymer like cellulose. Accordingly, the template of the method of the present invention comprises in one embodiment a water-insoluble matrix of mussel shell, snail shell or any other chitin containing protecting armament of a sea water animal or insect, a functionalized chitin thereof, or any other natural, functionalized polymer of any other source, preferably wherein the functionalized polymer is cellulose.

In one embodiment the template is prepared for the desired geometry of the mature synthetic nacre sample. More specifically, the template is preferably designed according to the geometry of the desired part, or designed according to the composition of the polymer fraction in mature nacre. Most preferably, the template is designed with tuneable pore size.

In a further preferred embodiment of the method of the present invention, the template is mounted on a device such as any kind of sample holder or any geometry.

To reach the outstanding mechanical properties of nacre like high fracture toughness and a favorable compliance it is essential that the mineral plates are densely packed between the organic sheets. Furthermore the mineral are joined together by the adhesive function of the organic matrix. This situation has also been calculated by finite element analysis (FEM, Fig. 3 and Fig. 4). The results of the FEM calculation reveal the dependence of the Young's modulus of the biocomposite nacre on the Poisson's ratio of the biopolymer. The high Young's modulus of the natural composite (near 80 GPa) has to be attributed to a high effective Poisson's ratio of the organic matrix (Fig. 4). In accordance with the present invention, the organic matrix plays a dominating role on the mechanical properties of nacre. The effect of additives, templates and numerous process parameters on the crystallization and consolidation of calcium carbonate has been investigated and recently reviewed (see for example Meldrum, Internat. Mater. Reviews 48,3 (2003), 187-223; Cölfen, Curr. Opin. Colloid a. Interface Science 8 (2003), 23-31), and the structural evolution of many different shapes and parts e.g. porous single crystals (see for example Zhan et al., Adv. Mater. 15,7-8 (2003), 621-623), and microparticles (see for example Sukhorukov et al., J. Mater. Chem. 14 (2004), 2073-2081) has been described. Much effort is also spent to the development of processes which address two objectives simultaneously: i.e. chemical conversion of biogenic species while the shape and some structural features of the original species are preserved. This approach led to the BaSIC-process (Biosculpting and Shape-preserving Inorganic Conversion); see for example Sandhage et al., Adv. Mater. 14 (2002), 429-433.

In accordance with the present invention the water-insoluble matrix of the sea water snail *Haliotis laevigata* could be shown to form a three dimensional template for the crystallization of aragonite tablets; see also the Examples. Therefore, in a preferred embodiment of the present invention the template comprises or consists of the water-insoluble matrix of nacre of the sea-water snail of any species of *Haliotis,* preferably the water-insoluble matrix of nacre of the sea-water snail *Haliotis laevigata.*

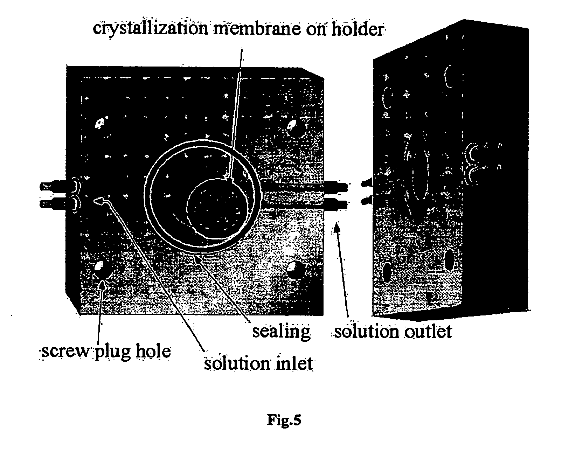

As discussed above and described in the Examples, the method of the present invention, the crystallization is preferably performed within a device which allows the template to be mounted such that the continuous flow of the calcium and carbonate, respectively, ion containing solution can take place on either side of the template for allowing double diffusion. This can be achieved for example with providing a corresponding compartment such as a box hollowed by the device. For the purposes of the present invention, such a compartment or box may be coined "double diffusion crystallization box", "double diffusion box" or "crystallization box". If the entire device is specifically meant, also the term "double diffusion device" may be used. The general setup for such a crystallization box is shown in Fig. 5. A piece of an ion permeable membrane is placed on an o-ring and then mounted inside the crystallization box. In the case of for example regenerated cellulose, a second o-ring has to be placed on the other side of the membrane as a spacer.

Accordingly, in a preferred embodiment of the invention, the device comprises a double diffusion crystallization box, or preferably it comprises a double diffusion crystallization box, a template by which the box when closed is separated into two separate halves, a first and a second half, a template-holder, and solution inlet(s) and outlet(s) in each of the two halves of the box, and optionally said device further comprises means for continuously pumping solutions through the two separated parts of the box, and optionally solution reservoirs. A similar crystallization device has been described in Becker and Epple, (2005); see supra. This crystallization device, in particular the crystallization box can be used and adapted in accordance with the teaching of the present invention.

The present invention also provides a device as defined supra, preferably a device comprising a double diffusion crystallization box which when closed is separated into two separate halves, a first and a second half by a membrane if present, a membrane-holder, solution inlet(s) and outlet(s) in each of the two halves of the box, optionally means for continuously pumping solutions through the two separated parts of the box and/or solution reservoirs.

In a particular preferred embodiment, the present invention relates to said device with the membrane present, most preferably wherein the membrane is a template as defined hereinbefore. In a particular preferred embodiment, said device comprises a membrane or template which comprises or consists of chitin or an equivalent polymer; see supra.

In addition, the present invention relates to a crystallization set up comprising one or more of the above-described crystallization devices or boxes, one container containing a calcium ion containing solution with a pH about 6.5 to 7.5, most preferably about 7, and one container containing a carbonate ion containing solution with a pH about 7.5 to 8.5, most preferably about 8, optionally pH meters, computer devices, one or more peristaltic pumps, and other means which allow the system to be used in the method of the present invention as described herein. Additional or alternative means for the device and set up of the present invention are described in the Examples. Insofar other means can be used for carrying out the above-described method, the present invention also relates to the use of any such device, crystallization box and set up for use in the methods as disclosed herein.

In a further aspect, the present invention relates to biogenic polymer/mineral composites and compositions comprising the same, obtainable by the novel method described herein and in the Examples. More particularly, the present invention relates to nacre and derivatives thereof obtainable by the method of the present invention. In this context, the final microstructure is preferably virtually identical to natural nacre. Sheets of platelets formed inside the insoluble matrix (fracture region displayed in Fig. 7). In Fig. 7 a region is displayed, where the insoluble matrix surface is cracked uncovering thin sheets of platelets and organic material.

However, the method of manufacturing biogenic polymer/mineral composites in accordance with the present invention has the additional advantage that the method can be performed such that resulting biomineral is free of components present in native crystals, which for example do not contribute to the desired properties of the biomineral. For example, inclusion bodies formed from substances in the seawater, in particular pollutants, or proteinaceous material such as proteins which are secreted by the animal which produces the biominerals such as nacre in nature may not be desired to be present in the biomineral, in particular if a medical application of the biomineral is envisaged. Accordingly, the biomaterial provided in accordance with the present invention, in particular nacre and derivatives thereof, can have virtually identical and even superior properties compared to their natural counterparts but are devoid of undesired substances. Thus, new synthetic biominerals can be custom designed and produced in accordance with the method of the present invention while realizing the idealized microstructure of native biominerals, in particular nacre, and its fascinating properties.

Accordingly, in a further important aspect the invention relates to synthetic biominerals, in particular "synthetic nacre". As mentioned, the synthetic biomineral can be distinguished from the corresponding native biomineral by determining the absence or at least less extent of components usually present in naturally grown biominerals due to environmental conditions. In particular, the present invention relates to "synthetic nacre" which can be distinguished from native nacre by several characteristics such as for example tuneable properties, or tuneable components, depending on the solutions used for crystallization. Furthermore, the synthetic nacre of the present invention can be manufactured in the absence of animals the presence of which is one requirement for naturally grown nacre. Moreover, the synthetic nacre may comprise tuneable mechanical and optical properties, thickness, size of structural components and as a further aspect tuneable grade of purity. Furthermore, it may comprise a tuneable organic/mineral ration and shape. In one embodiment, the synthetic biomineral is distinguished from the corresponding native biomineral by the substantially absence of one or more proteins or peptides usually present in the native biomineral. In an alternative or additional embodiment, the synthetic biomineral does not substantially comprise chitin. In a still further embodiment, the synthetic biomineral of the present invention comprises a component which is not found in a corresponding native biomineral, for example an artificial, synthetic or recombinant protein or peptide and/or an artificial or synthetic water-insoluble and/or hydrophobic matrix or sheet. In this context, the term "artificial" and "synthetic", respectively, describes the fact that the product so designated does not or cannot occur in nature but has to be produced in vitro, i.e. by man. In order to identify synthetic biomineral of the present invention a corresponding probe may be compared with the respective native biomineral, if present, and analyzed by means such as described herein and known to the person skilled in the art.

Three important features of the new material "synthetic nacre" are the soft, corrosion resistant water-insoluble organic matrix, the dense packing of the mineral plates and the interaction of the organic material with the mineral plates.

As already discussed with the general features of biominerals, it is evident to the person skilled in the art that there are various applications for the biogenic polymer/mineral compositions provided by the present invention, especially for nacre and for synthetic nacre in particular. For example, biominerals such as nacre find use as bioceramic as well as in decorating surfaces to mention only two major applications. Accordingly, the present invention also relates to compositions, devices, and any other product which comprises, for example on its surface, a biogenic polymer/mineral composition, preferably nacre.

As mentioned, those skilled in the art will appreciate/recognize, that there is a broad field of new future implementations for sustainable polymer/mineral composite materials of any geometry with tuneable high performance mechanical properties on the basis of biomineralization of nacre such as "anti-fouling" coatings for sea water proofed objects or instruments, since synthetic nacre can be used to substitute toxic antifouling paint used for hulls and other objects immersed in seawater to form biocompatible surface coatings, as well as multilayered coatings for surfaces of walls, buildings etc., since synthetic nacre can be used for coatings of surfaces, which are subject to contaminations (graffiti, exhaust films etc.). The multilayered structure, where thin mineral layers alternate with thin organic layers can be applied in plated form, and if necessary can be peeled off layer by layer, by acetic acid treatment many times leaving a clean surface behind.

A further field of application is the provision of non-brittle ceramics formed at ambient conditions, since the fracture and corrosion resistant synthetic nacre promises new material with high elasticity combined with an optimized structure.

In this context, the nacre of the invention can also be used as construction material, for example, synthetic nacre can be used for light weighted structural panel in buildings, parts in transport systems like aircraft and automobiles and other applications.

The nacre according to the present invention can be also used in/as bullet-proof ceramics, since/because synthetic nacre can be as durable as natural nacre. It can have the same structure, two thousand or more layers of thin (0.5µm) plates between even thinner (0.05µm) organic material. Duplicating the structural design leads to such strong materials, to create armor like bullet-proof material. The organic material will deform on slow or fast impact, will dissipate the energy and stop the intruding material.

Therefore, it is also utilizable in mechanical stable, flexible membranes, providing thin sheets of ultra strong material.

Another very important application area is the use of nacre as biomaterial for medical engineering. For example, synthetic nacre of the present invention can be used in the field of medicine technique as bone or dental implants. To date implants from non-ceramic material is in many cases not biocompatible. Up to date the non-ceramic material is favored because of the higher mechanical reliability in comparison to the brittle ceramics. This can be overcome by using the polymer/mineral composite "synthetic nacre", which would display in addition a more physiological surface quality. Furthermore, synthetic nacre as bone craft substitute can help to support the growth of new bone material in bone fractures.

A further application concerns the field of nanocomposites, where the nacre of the present invention can be used for/in/as tunable nanocomposites leading to sustainable material with surface property gradients.

Accordingly, paint, coatings for surfaces or coatings for medical devices like needles, syringes or pumps, ceramics, buildings, transportation systems like aircraft, ships and automobiles, and/or bone or dental implant comprising nacre of the present invention is also provided.

These and other embodiments are disclosed and encompassed by the description and examples of the present invention. Further literature concerning any one of the materials, methods, uses and compounds to be employed in accordance with the present invention may be retrieved from public libraries and databases, using for example electronic devices. For example the public database "Medline" may be utilized, which is hosted by the National Center for Biotechnology Information and/or the National Library of Medicine at the National Institutes of Health. Further databases and web addresses, such as those of the European Bioinformatics Institute (EBI), which is part of the European Molecular Biology Laboratory (EMBL) are known to the person skilled in the art and can also be obtained using internet search engines. An overview of patent information in biotechnology and a survey of relevant sources of patent information useful for retrospective searching and for current awareness is given in Berks, TIBTECH 12 (1994), 352-364.

The above disclosure generally describes the present invention. Several documents are cited throughout the text of this specification. Full bibliographic citations may be found at the end of the specification immediately preceding the claims. The contents of all cited references (including literature references, issued patents, published patent applications as cited throughout this application and manufacturer's specifications, instructions, etc.) are hereby expressly incorporated by reference; however, there is no admission that any document cited is indeed prior art as to the present invention.

The above disclosure generally describes the present invention. A more complete understanding can be obtained by reference to the following specific examples which are provided herein for purposes of illustration only and are not intended to limit the scope of the invention.

### EXAMPLES

The examples which follow further illustrate the invention, but should not be construed to limit the scope of the invention in any way.

### Example 1: Preparation of nacre pieces

Shells of the gastropod *Haliotis laevigata* (obtained from Abalone Experts, Laverton North, Victoria Australia, stored at 4° C) were cleaned with water and a brush. Calcitic outer parts of the shell were removed by sand blasting. The remaining nacreous shell was crushed with a hammer in pieces of approximately 2 cm diameter. The pieces were incubated for 2 min in a solution of 1:1 sodium hypochlorite (NaOCl, Merck) and ultra pure water (Milli - Q Academic, Millipore) to remove organic contaminations from the surface. Hypochlorite and dissolved organic compounds were removed by extensive washing with ultra pure water.

### Example 2: Demineralization of nacre

Dialysis tubes (Spectra/Por RC, MWCO 3500, Spectrum Laboratories) were prepared by heating up to 100°C in 3 mM ethylene diamine tetraacetic acid (EDTA, AppliChem) to remove the glycerol coating, inactivate proteases and eventual heavy metal contaminations. The clean nacre pieces were demineralized by dialysis against 100 mM EDTA with 0.02% sodium azide (NaN₃, Fluka) at pH 5 constantly stirring at 4° C. The outer solution was exchanged every 1 - 2 days. Complete removal of mineral parts was indicated by the absence of CO₂ bubbles after 20 days. After 30 days the demineralization dialysis was stopped. Total demineralization of the insoluble matrix was confirmed by light microscopy and scanning electron microscopy (SEM) of the surface and inner parts. For storage, the insoluble matrix was dialyzed against 10 mM sodium bicarbonate (NaHCO₃, Sigma) with 0.02% NaN₃ pH 8.4 at 4° C.

### Example 3: Crystallization experiments

Crystallization was performed using double diffusion across an ion permeable membrane. The reservoir solutions were constantly renewed with a peristaltic pump to keep concentrations and pH constant and prevent in solution crystallization (Fig. 5) Thereby membrane surface charges are kept constant and crystallization can occur at a constant rate directly on the membrane, possibly showing interactions. A *similar device* has been previously published; see for example Becker and Epple, mater. Res. Soc. Symp. Proc. 2005. 873E:K12.1.1-K12.1.10.

Two types of membranes were used: The insoluble matrix of nacre and regenerated cellulose dialysis membranes (Spectra/Por RC, MWCO 3500, Spectrum Laboratories, prepared as described above in the section "Demineralization of nacre"). Membrane pieces of 14mm diameter were created with a punch and placed on a neoprene ring of 14mm outer, 12mm inner diameter and 0.48mm height. In case of very thin membranes a second neoprene ring was placed on the other side of the membrane as a spacer. The two sides of the crystallization box were flown through by 20 mM CaCl₂ (pH 7.3) respectively 20 mM NaHCO₃ (pH 8.4) solutions with a speed of 0.5 ml/min/side up to a total volume of 480 ml/side (approx. 10 hours). At the end of the experiment the membranes were cut into halves and dried at room temperature on a glass cover slide with different sides to the top. As a control identically treated excess membrane pieces (except mineralization) were kept and investigated with SEM; see also Fig. 6 and 7, showing the successful growth of aragonite crystals.

### Example 4: Scanning electron microscopy (SEM)

For scanning electron microscopic investigations, the specimens were gold coated with a sputter coater (Emitech K550) and investigated at 20 kV using a Camscan Series 2 (Cambridge Instruments) SEM in secondary or back-scattered electron imaging mode.

It is apparent that the present invention provides a method of manufacturing crystals enabling a more focused, or faster generation of a desired crystal compared to crystal growth in nature. For the first time a method is provided which allows for controlled growth of a crystal, as well as control of the crystal's quality. Moreover, the method of the present invention is not restricted to nacre, since the choice of certain parameters such as the template with an appropriate pore size, suitable ionic solutions to allow crystallization, and thereby generating a desired crystal is applicable to a number of crystals intended to be grown. It is conceivable to use the method of the present invention for the generation of a variety of crystals, thereby overcoming the limitations, as exhibited for example by crystals which either grow only poorly or very slowly in nature, or the "quality" (purity) of which, when naturally grown, is a deficiency rendering those crystals inapplicable for sustainable industrial purposes. Those drawbacks can be overcome by manufacturing crystals using the method of the present invention.

## Claims

1. A method of manufacturing nacre or a derivative thereof comprising:
(a) providing a two- or three-dimensional template which is permeable for ions and macromolecules in double diffusion;
(b) subjecting the template to a continuous flow of a calcium ion containing solution on one side and a carbonate ion containing solution on the other side of the template;
under conditions allowing nucleating crystals, wherein said template comprises a water-insoluble matrix of mussel shell or snail shell or chitin or an equivalent polymer such as cellulose.

2. The method of claim 1, wherein said crystals are aragonite crystals.

3. The method of claim 1 or 2, wherein
(a) said calcium ion containing solution comprises a dissolved calcium salt with a solubility product higher than calcium carbonate; and/or
(b) said carbonate ion containing solution comprises a dissolved carbonate salt with a solubility product higher than calcium carbonate,

4. The method of claim 3, wherein said calcium salt is CaCl₂ and/or CaNO₃ and/or said carbonate is NaHCO₃ and/or Na₂CO₃.

5. The method of any one of claims 1 to 4, wherein said calcium ion containing solution has a pH about 7 and/or said carbonate ion containing solution has pH about 8.

6. The method of any one of claims 1 to 5, wherein the three-dimensional template is the water-insoluble matrix of nacre of the sea-water snail of *Haliotis.*

7. The method of claim 6, wherein said sea-water snail is *Haliotis laevigata.*

8. The method of any one of claims 1 to 7, wherein the template is mounted on a device.

9. The method of claim 8, wherein said device comprises a double diffusion crystallization box.

10. A device comprising a double diffusion crystallization box for nacre or a derivative thereof which when closed is separated into two separate halves, a first and a second half by a membrane, if present, a membrane-holder, solution inlet(s) and outlet(s) in each of the two halves of the box, optionally means for continuously pumping solutions through the two separated parts of the box and/or solution reservoirs, wherein said device comprises two flow channels on each halve.

11. Synthetic nacre or a derivative thereof obtainable by the method of any one of claims 1 to 9.

12. Synthetic nacre of claim 11, which is substantially devoid of at least one component of native nacre such as a protein, a peptide, or chitin.

13. Paint, coatings for surfaces or coatings for medical devices like needles, syringes or pumps, ceramics, buildings, transportation systems like aircraft, ships and automobiles, and/or bone or dental implant comprising nacre of claim 11 or 12.

14. Use of a device of claim 10 for the manufacturing of synthetic nacre or a derivative thereof.

## Patentansprüche

1. Ein Verfahren zum Herstellen von Perlmutter oder einem Derivat davon, beinhaltend:
(a) Bereitstellen eines zwei- oder drei-dimensionalen Templats, das in Doppeldiffusion durchlässig ist für Ionen und Makromoleküle;
(b) Unterziehen des Templats einem kontinuierlichen Strom einer Calciumionen enthaltenden Lösung auf einer Seite, und einer Carbonationen enthaltenden Lösung auf der anderen Seite des Templats;
unter Bedingungen, die Keimbildung von Kristallen zulassen, wobei das Templat eine wasser-unlösliche Matrix von Muschelschale oder Schneckenschale oder Chitin oder einem äquivalenten Polymer, wie beispielsweise Zellulose, enthält.

2. Das Verfahren nach Anspruch 1, wobei die Kristalle Aragonitkristalle sind.

3. Das Verfahren nach Anspruch 1 oder 2, wobei
(a) die Calciumionen enthaltende Lösung ein gelöstes Calciumsalz mit einem Löslichkeitsprodukt enthält, das größer ist als Calciumcarbonat; und/oder
(b) die Carbonationen enthaltende Lösung ein gelöstes Carbonatsalz mit einem Löslichkeitsprodukt enthält, das größer ist als Calciumcarbonat.

4. Das Verfahren nach Anspruch 3, wobei das Calciumsalz CaCl₂ und/oder CaNO₃ und/oder das Carbonat NaHCO₃ und/oder Na₂CO₃ ist.

5. Das Verfahren nach einem der Ansprüche 1 bis 4, wobei die Calciumionen enthaltende Lösung einen pH von etwa 7 aufweist und/oder die Carbonationen enthaltende Lösung einen pH von etwa 8 aufweist.

6. Das Verfahren nach einem der Ansprüche 1 bis 5, wobei das drei-dimensionale Templat die wasser-unlösliche Matrix von Perlmutter der Meerwasserschnecke *Haliotis* ist.

7. Das Verfahren nach Anspruch 6, wobei die Meerwasserschnecke *Haliotis laevigata* ist.

8. Das Verfahren nach einem der Ansprüche 1 bis 7, wobei das Templat auf einer Vorrichtung befestigt ist.

9. Das Verfahren nach Anspruch 8, wobei die Vorrichtung ein Doppeldiffusions-Kristallisationsbehältnis aufweist.

10. Eine Vorrichtung aufweisend ein Doppeldiffusions-Kristallisationsbehältnis für Perlmutter oder einem Derivat davon, das im geschlossenen Zustand in zwei getrennte Hälften getrennt ist, eine erste und eine zweite Hälfte durch eine Membran, sofern vorhanden, eine Membran-Halterung, Lösungs-Einlassöffnung(en) und -Auslassöffnung(en) in jeder der zwei Hälften des Behältnisses, wahlweise Mittel zum kontinuierlichen Pumpen von Lösungen durch die zwei getrennten Teile des Behältnisses und/oder Lösungs-Reservoirs, wobei die Vorrichtung zwei Fließkanäle auf jeder Hälfte aufweist.

11. Synthetische Perlmutter oder ein Derivat davon, erhältlich durch das Verfahren nach einem der Ansprüche 1 bis 9.

12. Synthetische Perlmutter nach Anspruch 11, das im Wesentlichen frei ist von wenigstens einer Komponente von nativer Perlmutter, wie beispielsweise einem Protein, einem Peptid, oder Chitin.

13. Farbe, Beschichtungen für Oberflächen oder Beschichtungen für medizinische Vorrichtungen, wie zum Beispiel Nadeln, Spritzen oder Pumpen, Keramiken, Gebäude, Transportsysteme, wie zum Beispiel Flugzeuge, Schiffe und Automobile, und/oder Knochen oder Dentalimplantat, die Perlmutter nach Anspruch 11 oder 12 enthalten.

14. Verwendung einer Vorrichtung nach Anspruch 10 für die Herstellung von synthetischem Perlmutter oder einem Derivat davon.

## Revendications

1. Procédé de fabrication de nacre ou d'un dérivé de celle-ci, comprenant:
(a) la fourniture d'une matrice bi- ou tri-dimensionnelle qui est perméable aux ions et aux macromolécules en double diffusion ;
(b) la soumission de la matrice à un flux continu d'une solution contenant des ions calcium sur un côté, et à une solution contenant des ions carbonate sur l'autre côté de la matrice ;
sous des conditions permettant la nucléation de cristaux, dans lequel ladite matrice comprend une matrice insoluble dans l'eau faite de coquille de moule ou d'escargot ou de chitine ou d'un polymère équivalent tel que la cellulose.

2. Procédé selon la revendication 1, dans lequel lesdits cristaux sont des cristaux d'aragonite.

3. Procédé selon la revendication 1 ou 2, dans lequel
(a) ladite solution contenant des ions calcium comprend un sel de calcium dissous ayant un produit de solubilité supérieur au carbonate de calcium ; et/ou
(b) ladite solution contenant des ions carbonate comprend un carbonate dissous ayant un produit de solubilité supérieur au carbonate de calcium.

4. Procédé selon la revendication 3, dans lequel ledit sel de calcium est CaCl₂ et/ou CaNO₃ et/ou ledit carbonate est NaHCO₃ et/ou Na₂CO3.

5. Procédé selon l'une quelconque des revendications 1 à 4, dans lequel ladite solution contenant des ions calcium a un pH d'environ 7 et/ou ladite solution contenant des ions carbonate a un pH d'environ 8.

6. Procédé selon l'une quelconque des revendications 1 à 5, dans lequel la matrice tridimensionnelle est la matrice insoluble dans l'eau de nacre du mollusque marin *Haliotis.*

7. Procédé selon la revendication 6, dans lequel ledit mollusque marin est *Haliotis laevigata.*

8. Procédé selon l'une quelconque des revendications 1 à 7, dans lequel la matrice est montée sur un dispositif.

9. Procédé selon la revendication 8, dans lequel ledit dispositif comprend une boîte de cristallisation à double diffusion.

10. Procédé comprenant une boîte de cristallisation à double diffusion pour la nacre ou un dérivé de celle-ci qui, lorsqu'elle est fermée, est séparée en deux moitiés distinctes, une première et une seconde moitié, par une membrane, si présente un porte-membrane, une(des) entrée(s) et sortie(s) pour les solutions dans chacune des deux moitiés de la boîte, facultativement des moyens pour pomper en continu les solutions à travers les deux parties séparées de la boîte et/ou des réservoirs pour les solutions, dans lequel ledit dispositif comprend deux canaux d'écoulement sur chaque moitié.

11. Nacre synthétique ou dérivé de celle-ci pouvant être obtenu€ par le procédé selon l'une quelconque des revendications 1 à 9.

12. Nacre synthétique selon la revendication 11, qui est essentiellement dépourvue d'au moins un composant de la nacre native, tel qu'une protéine, un peptide ou la chitine.

13. Peintures, revêtements pour des surfaces ou des revêtements de dispositifs médicaux tels qu'aiguilles, seringues ou pompes, de céramiques, de bâtiments, de systèmes de transport tels qu'aéronefs, navires ou automobiles, et/ou des implants osseux ou dentaires, comprenant de la nacre selon la revendication 11 ou 12.

14. Utilisation d'un dispositif selon la revendication 10 pour la fabrication de nacre synthétique ou d'un dérivé de celle-ci.
